# EUROPEAN PATENT APPLICATION

(11) **EP 4 582 553 A1**
(43) Date of publication of application: **09.07.2025**
(21) Application number: 22957270.6
(22) Date of filing: 30.12.2022
(51) Int. Cl.: C12Q 1/68, C07K 16/40, C12Q 1/6851, C12N 15/11

(54) **ONE-TUBE RT-QPCR FULLY PREMIXED REACTION REAGENT, USE, AND RT-QPCR METHOD**

(30) Priority: 30.08.2022 CN 202211045852
(71) Applicant: Shanghai Biogerm Medical Technology Co., Ltd., Shanghai 201415 (CN)
(72) Inventor: GU, Dongdong, Shanghai 201415 (CN); ZHAO, Baihui, Shanghai 201415 (CN); MA, Binheng, Shanghai 201415 (CN); HOU, Qiaoli, Shanghai 201415 (CN)
(74) Representative: TBK
(86) International application number: PCT/CN2022/144290
(87) International publication number: WO 2024/045461

(57) **Abstract**

The present invention relates to a one-tube RT-qPCR fully premixed reaction reagent, use, an RT-qPCR method, and a one-tube rapid RT-qPCR fully premixed reaction system in the field of reverse transcription fluorescence PCR technology. The present invention aims at three problems in existing RT-qPCR reaction systems: (1) The total reaction system is divided into multiple tubes for storage, requiring components to be freshly prepared and used each time. Primers and probes must be stored separately or together with an amplification reaction solution. Pre-mixing the components in advance may lead to stability and specificity problems in the reaction system. (2) The RT-qPCR reaction system cannot adapt to rapid amplification programs, resulting in excessively long reaction times and unsuitability for rapid detection. (3) The compatibility between the RT-qPCR reaction system and the primers/probes is insufficient, necessitating adjustments to the formulation of the reaction system according to different primer-probe combinations.

## Description

### CROSS-REFERENCE TO RELATED APPLICATION(S)

This disclosure claims priority to Chinese Patent Application CN202211045852.3 entitled "ONE-TUBE RT-QPCR FULLY PREMIXED REACTION REAGENT, USE, AND RT-QPCR METHOD" filed with the China National Intellectual Property Administration (CNIPA) on Aug. 30, 2022, the disclosure of which is incorporated herein by reference in its entirety.

### TECHNICAL FIELD

The present disclosure relates to the field of reverse transcription fluorescence PCR technology and, in particular, to a one-tube RT-qPCR fully premixed reaction reagent, a use and an RT-qPCR method.

### BACKGROUND

Polymerase chain reaction (PCR) is a method for performing rapid in-vitro amplification on specific DNA fragments using a single-stranded nucleotide primer. The reaction is an exponential reaction that can amplify a very tiny amount of target DNA fragments by millions of times in a short period of time.

RT-qPCR is a method in which reverse transcriptases are added on the basis of PCR and RNA is used as an original template for amplification to obtain DNA. RT-qPCR monitors an amplification product in each cycle of a PCR reaction in real-time according to a change in the intensity of fluorescent signals accumulated by fluorescent groups added to the reaction system.

At present, a commonly used RT-qPCR reaction system consists of a nucleic acid amplification buffer, an enzyme-mixed solution and a primer probe reaction solution and needs to be stored separately. When the reaction system is used, each component needs to be dissolved at room temperature and thoroughly and homogeneously mixed for later use. When the reaction system is used, each component is added according to a fixed proportion and homogeneously mixed before being subpackaged into a PCR tube. An entire process needs calculation and careful operation, and wrong preparation often occurs. Moreover, the reaction system needs to be prepared and used in site. If the nucleic acid amplification buffer, the enzyme-mixed solution and the primer probe reaction solution are premixed in advance, the stability problem will occur after the reaction system is frozen or cryopreserved and is specifically manifested as decreased sensitivity and non-specific amplification in negative samples.

The amplification efficiency of primer probes of the RT-qPCR reaction system in a rapid program is directly related to the nucleic acid amplification buffer and the enzyme-mixed solution in the reaction system. Generally, a group of nucleic acid amplification buffer and enzyme-mixed solution needs to be developed in correspondence to a group of primer probes, and primer probes cannot be shared between reaction systems. The RT-qPCR reaction system has relatively poor applicability to various primer probe reaction solutions.

### SUMMARY

The present disclosure provides a one-tube RT-qPCR fully premixed reaction reagent. The reagent includes a Taq enzyme monoclonal antibody with a double blocking ability.

Optionally, an amino acid sequence of the Taq enzyme monoclonal antibody with the double blocking ability is shown in SEQ ID No.1 and SEQ ID No.2.

Optionally, a content ratio (U/U) of the Taq enzyme monoclonal antibody with the double blocking ability to a Taq DNA polymerase is 1:3-9, preferably 1:5.

Optionally, the reagent further includes a nucleic acid aptamer.

Optionally, the number of bases contained in the nucleic acid aptamer is 50-100 bp.

Optionally, the nucleic acid aptamer has a concentration of 0.2-0.7 ng/mL. Optionally, the nucleic acid aptamer has a concentration of 0.5 ng/mL.

Optionally, the reagent further includes a stabilizer and/or an enhancer.

Optionally, the stabilizer includes at least two of 0.5-1 mg/mL BSA, 0.005% to 0.008% (v/v) gelatin, 5% to 10% (v/v) glycerol or a 0.1% to 0.2% (v/v) non-ionic detergent.

Optionally, the stabilizer includes 0.7 mg/mL BSA, 0.006% (v/v) gelatin and 7% (v/v) glycerol.

Optionally, the enhancer includes 1% to 2% (v/v) formamide, 1% to 5% (v/v) DMSO, 0.1-0.3 M betaine, a 0.5-1.0 g/L SSB (single-stranded DNA binding protein), a 0.3-0.8 g/L pyrophosphatase, 0.1-0.3 M trehalose and 0.05-0.1 g/L sodium azide.

Optionally, the enhancer includes 2% formamide, 2.5% DMSO, 0.2 M betaine, a 0.8 g/L SSB (single-stranded DNA binding protein), 0.2 M trehalose and 0.08 g/L sodium azide.

The present disclosure further provides a use of any one of the one-tube RT-qPCR fully premixed reaction reagents described above for preparing a one-tube RT-qPCR fully premixed reaction system.

The present disclosure further provides a one-tube RT-qPCR fully premixed reaction system. The system includes any one of the one-tube RT-qPCR fully premixed reaction reagents described above.

The system further includes a DNA polymerase, a reverse transcriptase, an UNG enzyme, an RNase inhibitor, a buffer, a stabilizer, an enhancer, upstream and downstream primers and a fluorescently labeled probe composition.

Optionally, the DNA polymerase is a hot start Taq DNA polymerase with an extension rate of 1.5 sec/kb and a preferred addition amount of 3-6 U.

Optionally, the reverse transcriptase includes an M-MLV reverse transcriptase with a tolerance temperature of more than or equal to 60 °C and an addition amount of 5-10 U.

Optionally, the UNG enzyme is 0.1-0.5 U thermosensitive UNG enzyme.

Optionally, an addition amount of the RNase inhibitor is 2-3 U.

Optionally, the buffer is a 100-500 mmol/L Tris-HCl buffer solution.

Optionally, the buffer contains an inorganic salt, where the inorganic salt is a sulfate and/or a chloride salt. Optionally, the inorganic salt is the chloride salt.

Optionally, the upstream and downstream primers and fluorescently labeled probe composition include a single detection composition, a double detection composition, a triple detection composition or a quadruple detection composition based on a difference in the number of types of a nucleic acid product obtained after amplification in a single time.

Optionally, the nucleic acid product is derived from a virus.

Optionally, the virus is selected from at least one of norovirus, influenza A virus, influenza B virus, enteric conventional virus, Enterovirus 71 or Coxsackievirus 16.

Optionally, the single detection composition is a primer probe composition of norovirus.

Optionally, a nucleotide sequence of an upstream primer in the primer probe composition of norovirus is shown in SEQ ID No.3, a nucleotide sequence of a downstream primer in the primer probe composition of norovirus is shown in SEQ ID No.4, and a nucleotide sequence of a probe in the primer probe composition of norovirus is shown in SEQ ID No.5.

Optionally, the double detection composition is a primer probe composition of influenza A virus and influenza B virus.

Optionally, in the primer probe composition of influenza A virus and influenza B virus, a nucleotide sequence of an upstream primer of influenza A virus is shown in SEQ ID No.6, a nucleotide sequence of a downstream primer of influenza A virus is shown in SEQ ID No.7, a nucleotide sequence of a probe of influenza A virus is shown in SEQ ID No.8, a nucleotide sequence of an upstream primer of influenza B virus is shown in SEQ ID No.9, a nucleotide sequence of a downstream primer of influenza B virus is shown in SEQ ID No.10, and a nucleotide sequence of a probe of influenza B virus is shown in SEQ ID No.11.

Optionally, the triple detection composition is a primer probe composition of enteric conventional virus, Enterovirus 71 and Coxsackievirus 16.

Optionally, the primer probe composition of enteric conventional virus, Enterovirus 71 and Coxsackievirus 16 contains:
a universal upstream primer of enteric conventional virus with a nucleotide sequence shown in SEQ ID No.12, a universal downstream primer of enteric conventional virus with a nucleotide sequence shown in SEQ ID No.13 and a universal probe of enteric conventional virus with a nucleotide sequence shown in SEQ ID No. 14;
an upstream primer of Enterovirus 71 with a nucleotide sequence shown in SEQ ID No.15, a downstream primer of Enterovirus 71 with a nucleotide sequence shown in SEQ ID No.16 and a probe of Enterovirus 71 with a nucleotide sequence shown in SEQ ID No.17; and
an upstream primer of Coxsackievirus 16 with a nucleotide sequence shown in SEQ ID No.18, a downstream primer of Coxsackievirus 16 with a nucleotide sequence shown in SEQ ID No.19 and a probe of Coxsackievirus 16 with a nucleotide sequence shown in SEQ ID No.20.

The present disclosure further provides an RT-qPCR method. The method includes: amplifying a to-be-detected sample, by using the one-tube RT-qPCR fully premixed reaction system described above, according to the following program: reverse transcription for 1-5 min at 55-65 °C, predenaturation for 5-15s at 94-98 °C, denaturation for 1-5s at 94-98 °C, annealing and extension for 8-15s at 55-65 °C, and 38 to 45 cycles of denaturation, annealing and extension.

Optionally, the program of amplification includes: reverse transcription for 2 min at 60 °C, predenaturation for 10s at 95 °C, denaturation for 1s at 95 °C, annealing for 10s at 60 °C, extension for 10s at 60 °C, and 40 cycles of denaturation, annealing and extension.

The present disclosure further provides a use of any one of the one-tube RT-qPCR fully premixed reaction reagents described above or any one of the one-tube RT-qPCR fully premixed reaction systems described above for viral infection diagnosis.

Optionally, the virus includes at least one of norovirus, influenza A virus, influenza B virus, enteric conventional virus, Enterovirus 71 or Coxsackievirus 16.

The present disclosure further provides a method for diagnosing a disease related to viral infection in a subject. The method includes:
(A) performing amplification on a sample from the subject through an RT-qPCR method under a condition sufficient for a binding reaction to occur, with any one of the one-tube RT-qPCR fully premixed reaction reagents described above or any one of the one-tube RT-qPCR fully premixed reaction systems described above; and
(B) determining a viral origin of an amplification product.

Optionally, the virus includes at least one of norovirus, influenza A virus, influenza B virus, enteric conventional virus, Enterovirus 71 or Coxsackievirus 16.

Optionally, a source of the sample is selected from feces, vomit, a nasal swab, a throat swab, sputum, a bronchoalveolar lavage fluid, cells, chick embryo culture, a herpetic fluid, serum, a cerebrospinal fluid or tissue culture.

### BRIEF DESCRIPTION OF DRAWINGS

To illustrate the technical solutions in specific embodiments of the present disclosure or the technical solutions in the existing art more clearly, drawings used in the description of the specific embodiments or the existing art will be briefly described below. Apparently, the drawings described below illustrate part of the embodiments of the present disclosure, and those skilled in the art may obtain other drawings based on the drawings described below on the premise that no creative work is done.
FIG. 1 illustrates amplification results of a fully premixed RT-PCR reaction system and a control group in Example 1 of the present disclosure.
FIG. 2 illustrates amplification results of a fully premixed RT-PCR reaction system and a control group in Example 2 of the present disclosure.
FIG. 3 illustrates amplification results of fully premixed RT-PCR reaction systems and control groups in Example 2 of the present disclosure.
FIG. 4 illustrates amplification results of fully premixed RT-PCR reaction systems and control groups in Example 2 of the present disclosure.
FIG. 5 illustrates amplification results of a fully premixed RT-PCR reaction system and a control group in Example 3 of the present disclosure.
FIG. 6 illustrates amplification results of a fully premixed RT-PCR reaction system and a control group in Example 4 of the present disclosure.
FIG. 7 illustrates amplification results of a fully premixed RT-PCR reaction system and a control group in Example 5 of the present disclosure.
FIG. 8 illustrates amplification results of a fully premixed RT-PCR reaction system and a control group in Example 6 of the present disclosure.
FIG. 9 illustrates long-term stability detection results of a fully premixed RT-PCR reaction system in Example 6 of the present disclosure after the reaction system is stored for one month.
FIG. 10 illustrates long-term stability detection results of a fully premixed RT-PCR reaction system in Example 6 of the present disclosure after the reaction system is stored for three months.
FIG. 11 illustrates long-term stability detection results of a fully premixed RT-PCR reaction system in Example 6 of the present disclosure after the reaction system is stored for six months.
FIG. 12 illustrates long-term stability detection results of a fully premixed RT-PCR reaction system in Example 6 of the present disclosure after the reaction system is stored for nine months.
FIG. 13 illustrates long-term stability detection results of a fully premixed RT-PCR reaction system in Example 6 of the present disclosure after the reaction system is stored for twelve months.
FIG. 14 illustrates long-term stability detection results of a fully premixed RT-PCR reaction system in Example 6 of the present disclosure after the reaction system is stored for fifteen months.

### DETAILED DESCRIPTION

To illustrate the object, technical solutions and advantages in embodiments of the present disclosure more clearly, the technical solutions in the embodiments of the present disclosure will be described clearly and completely in conjunction with drawings in the embodiments of the present disclosure. Apparently, the embodiments described below are part, not all, of embodiments of the present disclosure. Generally, the components in the embodiments of the present disclosure described and illustrated in the drawings herein may be arranged and designed through various configurations.

Therefore, the following detailed description of the embodiments of the present disclosure provided in the drawings is not intended to limit the scope of the present disclosure, but merely represents selected embodiments of the present disclosure. Based on the embodiments described herein, all other embodiments obtained by those of ordinary skill in the art without creative work are within the scope of the present disclosure.

### Definition of terms

As used herein, terms "subject", "individual" and "patient" are interchangeable herein and refer to vertebrates, preferably mammals, and most preferably humans. Mammals include, but are not limited to, mice, apes, humans, livestock, animals in sport and pets. Tissues, cells and progenies of the tissues and the cells of biological entities obtained in vivo or cultured in vitro are also included.

As used herein, term "primer" refers to an oligonucleotide. The oligonucleotide can serve as a starting point of synthesis when placed under a condition of triggering the synthesis of a primer extension product complementary to a nucleic acid strand (in the presence of a nucleotide and an inducing substance (for example, a DNA polymerase) and at an appropriate temperature and pH). The primer may be single-stranded and must be long enough to trigger the synthesis of the needed extension product in the presence of the inducing substance. An exact length of the primer depends on numerous factors including a temperature, a source of the primer and a use of a method.

As used herein, term "diagnosis" or "medical diagnosis" refers to a judgment made on peoples' mental and physical states from a medical perspective. Specifically, "diagnosis" or "medical diagnosis" is a process of determining which disease or condition can explain symptoms and signs of a subject. For example, the one-tube RT-qPCR fully premixed reaction reagent or the one-tube RT-qPCR fully premixed reaction system described herein is used for determining the presence of a disease related to viral infection in the subject.

An embodiment of the present disclosure provides a one-tube RT-qPCR fully premixed reaction reagent. The reagent includes a Taq enzyme monoclonal antibody with a double blocking ability.

In an optional embodiment, an amino acid sequence of the Taq enzyme monoclonal antibody with the double blocking ability is shown in SEQ ID No.1 (DIEMTQSSGAELARPGASVKMSCKASGTFRYSDYMHWVKQRPGQGLEWIGYRGPNDR YTKYNQKFKKATLTADKSSSTAYMQLSSLTSEDSAVYYCGRDDYYCSHDATWGQGTTLS RT) and SEQ ID No.2 (VQLVESGGMSASPGEKVTMTCAASSVYSSMHWYQQKSGTSPKRWIYDYQKLASGVPA RFSGSGSGTSYSLTISSMEAETPETYYCEDWNPSDTFFGSGTTISRDN).

In an optional embodiment, a content ratio (U/U) of the Taq enzyme monoclonal antibody with the double blocking ability to a Taq DNA polymerase is 1:3-9 and may be, for example, 1:3.5-8.5, 1:4-8 or 1:5-7, for example, 1:3, 1:4, 1:5, 1:6, 1:7, 1:8 or 1:9, or an interval value between any two end point values. Optionally, the content ratio (U/U) of the Taq enzyme monoclonal antibody with the double blocking ability to the Taq DNA polymerase is 1:5.

In an optional embodiment, the reagent further includes a nucleic acid aptamer.

In an optional embodiment, the number of bases contained in the nucleic acid aptamer is 50-100 bp, for example, 55-90 bp, 65-80 bp or 75-80 bp, for example, 50 bp, 60 bp, 70 bp, 80 bp, 90 bp or 100 bp, or an interval value between any two end point values.

Optionally, the nucleic acid aptamer has a concentration of 0.2-0.7 ng/mL, for example, 0.2 ng/mL, 0.3 ng/mL, 0.4 ng/mL, 0.5 ng/mL, 0.6 ng/mL or 0.7 ng/mL, or an interval value between any two end point values. Optionally, the nucleic acid aptamer has a concentration of 0.5 ng/mL.

In an optional embodiment, the reagent further includes a stabilizer and/or an enhancer.

Optionally, the stabilizer includes at least two of 0.5-1 mg/mL BSA, 0.005% to 0.008% (v/v) gelatin, 5% to 10% (v/v) glycerol or a 0.1% to 0.2% (v/v) non-ionic detergent. For example, a content of BSA is 0.5 mg/mL, 0.6 mg/mL, 0.7 mg/mL, 0.8 mg/mL, 0.9 mg/mL or 1 mg/mL, or an interval value between any two end point values. For example, a content (v/v) of gelatin is 0.005%, 0.006%, 0.007% or 0.008%, or an interval value between any two end point values. For example, a content (v/v) of glycerol is 5%, 6%, 7%, 8%, 9% or 10%, or an interval value between any two end point values. For example, a content (v/v) of the non-ionic detergent is 0.1%, 0.14%, 0.18% or 0.2%, or an interval value between any two end point values.

Optionally, the stabilizer includes 0.7 mg/mL BSA, 0.006% (v/v) gelatin and 7 % (v/v) glycerol.

Optionally, the enhancer includes 1% to 2% (v/v) formamide, 1% to 5% (v/v) DMSO, 0.1-0.3 M betaine, a 0.5-1.0 g/L SSB (single-stranded DNA binding protein), a 0.3-0.8 g/L pyrophosphatase, 0.1-0.3 M trehalose and 0.05-0.1 g/L sodium azide. For example, a content (v/v) of formamide is 1%, 1.5% or 2%, or an interval value between any two end point values. For example, a content (v/v) of DMSO is 1%, 2%, 3%, 4% or 5%, or an interval value between any two end point values. For example, a content of betaine is 0.1 M, 0.2 M or 0.3 M, or an interval value between any two end point values. For example, a content of the SSB (single-stranded DNA binding protein) is 0.5 g/L, 0.7 g/L, 0.9 g/L or 1.0 g/L, or an interval value between any two end point values. For example, pyrophosphatase has a content of 0.3 g/L, 0.4 g/L, 0.5 g/L, 0.6 g/L, 0.7 g/L or 0.8 g/L, or an interval value between any two end point values. For example, a content of trehalose may be 0.1 M, 0.2 M or 0.3 M, or an interval value between any two end point values. For example, a content of sodium azide may be 0.05 g/L, 0.06 g/L, 0.07 g/L, 0.08 g/L, 0.09 g/L or 1.0 g/L, or an interval value between any two end point values.

Optionally, the enhancer includes 2% formamide, 2.5% DMSO, 0.2 M betaine, a 0.8 g/L SSB (single-stranded DNA binding protein), 0.2 M trehalose and 0.08 g/L sodium azide.

An embodiment of the present disclosure further provides a use of the one-tube RT-qPCR fully premixed reaction reagent in any one of the preceding embodiments for preparing a one-tube RT-qPCR fully premixed reaction system.

An embodiment of the present disclosure further provides a one-tube RT-qPCR fully premixed reaction system. The system includes the one-tube RT-qPCR fully premixed reaction reagent in any one of the preceding embodiments.

The system further includes a DNA polymerase, a reverse transcriptase, an UNG enzyme, an RNase inhibitor, a buffer, a stabilizer, an enhancer, upstream and downstream primers and a fluorescently labeled probe composition.

Optionally, the DNA polymerase is a hot start Taq DNA polymerase with an extension rate of 1.5 sec/kb. Optionally, the DNA polymerase has an addition amount of 3-6 U, for example, 3 U, 4 U, 5 U or 6 U, or an interval value between any two end point values.

Optionally, the reverse transcriptase includes an M-MLV reverse transcriptase with a tolerance temperature of more than or equal to 60 °C and an addition amount of 5-10 U.

Optionally, the UNG enzyme is 0.1-0.5 U (for example, 0.1 U, 0.2 U, 0.3 U, 0.4 U or 0.5 U, or an interval value between any two end point values) thermosensitive UNG enzyme.

Optionally, an addition amount of the RNase inhibitor is 2-3 U.

Optionally, the buffer is a 100-500 mmol/L Tris-HCl buffer solution.

Optionally, the buffer contains an inorganic salt, wherein the inorganic salt is a sulfate and/or a chloride salt, optionally the chloride salt.

In an optional embodiment, the upstream and downstream primers and fluorescently labeled probe composition include a single detection composition, a double detection composition, a triple detection composition or a quadruple detection composition based on a difference in the number of types of a nucleic acid product obtained after amplification in a single time.

Optionally, the nucleic acid product is derived from a virus.

Optionally, the virus is selected from at least one of norovirus, influenza A virus, influenza B virus, enteric conventional virus, Enterovirus 71 or Coxsackievirus 16.

Optionally, the single detection composition is a primer probe composition of norovirus.

Optionally, a nucleotide sequence of an upstream primer in the primer probe composition of norovirus is shown in SEQ ID No.3 (CTGGTGGATTGGAAATGTA), a nucleotide sequence of a downstream primer in the primer probe composition of norovirus is shown in SEQ ID No.4 (AATTCGGGCAGAAGATTG), and a nucleotide sequence of a probe in the primer probe composition of norovirus is shown in SEQ ID No.5 (CTGTCCACAATCCGAGGTCAT).

Optionally, the double detection composition is a primer probe composition of influenza A virus and influenza B virus.

Optionally, in the primer probe composition of influenza A virus and influenza B virus, a nucleotide sequence of an upstream primer of influenza A virus is shown in SEQ ID No.6 (AGTCTTCTAACCGAGGTC), a nucleotide sequence of a downstream primer of influenza A virus is shown in SEQ ID No.7 (TCGAGATCTGCGTTCTTC), a nucleotide sequence of a probe of influenza A virus is shown in SEQ ID No.8 (CATCCTCAAGTCTCTGTGCGA), a nucleotide sequence of an upstream primer of influenza B virus is shown in SEQ ID No.9 (GGAGTCTTATCCCAATTTG), a nucleotide sequence of a downstream primer of influenza B virus is shown in SEQ ID No.10 (CTTCTGTGACCAGTCTAA), and a nucleotide sequence of a probe of influenza B virus is shown in SEQ ID No.11 (CAAGAGCACCGATTATCACCAGAA).

Optionally, the triple detection composition is a primer probe composition of enteric conventional virus, Enterovirus 71 and Coxsackievirus 16.

Enteric conventional virus includes poliovirus, Coxsackievirus and orphan virus (ECHO virus) that causes a cytopathic effect in a human gut. In 1970, the International Committee on Nomenclature of Viruses classified these viruses as the genus Enterovirus within the family Picornaviridae.

Optionally, the primer probe composition of enteric conventional virus, Enterovirus 71 and Coxsackievirus 16 contains:
a universal upstream primer of enteric conventional virus with a nucleotide sequence shown in SEQ ID No.12 (GCAAGTAGTCACAGATTAG), a universal downstream primer of enteric conventional virus with a nucleotide sequence shown in SEQ ID No.13 (GATTCTTGTCACTAGCATT) and a universal probe of enteric conventional virus with a nucleotide sequence shown in SEQ ID No.14 (TACCAGCACTACAAGCCGC);
an upstream primer of Enterovirus 71 with a nucleotide sequence shown in SEQ ID No.15 (CACAGGTGAGCAGTCATC), a downstream primer of Enterovirus 71 with a nucleotide sequence shown in SEQ ID No.16 (CGCCCTACTGAAGAAACT) and a probe of Enterovirus 71 with a nucleotide sequence shown in SEQ ID No.17 (TACAGGCAAGGTTCCAGCAC); and
an upstream primer of Coxsackievirus 16 with a nucleotide sequence shown in SEQ ID No.18 (CTTCCAGAGATTCGTTTG), a downstream primer of Coxsackievirus 16 with a nucleotide sequence shown in SEQ ID No.19 (CATATTATTCGGGCATTGA) and a probe of Coxsackievirus 16 with a nucleotide sequence shown in SEQ ID No.20 (CAGACAGCCACCAACCCAT).

An embodiment of the present disclosure further provides an RT-qPCR method. The method includes: amplifying a to-be-detected sample, by using the one-tube RT-qPCR fully premixed reaction system in the preceding embodiment, according to the following program: reverse transcription for 1-5 min at 55-65 °C, predenaturation for 5-15s at 94-98 °C, denaturation for 1-5s at 94-98 °C, annealing and extension for 8-15s at 55-65 °C, and 38 to 45 cycles of denaturation, annealing and extension.

Optionally, the program of amplification includes: reverse transcription for 2 min at 60 °C, predenaturation for 10s at 95 °C, denaturation for 1s at 95 °C, annealing for 10s at 60 °C, extension for 10s at 60 °C, and 40 cycles of denaturation, annealing and extension.

An embodiment of the present disclosure further provides a use of the one-tube RT-qPCR fully premixed reaction reagent in any one of the preceding embodiments, the one-tube RT-qPCR fully premixed reaction system in any one of the preceding embodiments or the RT-qPCR method in the preceding embodiment for viral infection diagnosis.

An embodiment of the present disclosure further provides a use of the one-tube RT-qPCR fully premixed reaction reagent in any one of the preceding embodiments or the one-tube RT-qPCR fully premixed reaction system in any one of the preceding embodiments for viral infection diagnosis.

Optionally, the virus includes at least one of norovirus, influenza A virus, influenza B virus, enteric conventional virus, Enterovirus 71 or Coxsackievirus 16.

An embodiment of the present disclosure further provides a method for diagnosing a disease related to viral infection in a subject. The method includes:
(A) performing amplification on a sample from the subject through an RT-qPCR method under a condition sufficient for a binding reaction to occur, with the one-tube RT-qPCR fully premixed reaction reagent in any one of the preceding embodiments or the one-tube RT-qPCR fully premixed reaction system in any one of the preceding embodiments; and
(B) determining a viral origin of an amplification product.

An embodiment of the present disclosure further provides a method for diagnosing viral infection. The diagnosis method includes: preparing, by using the one-tube RT-qPCR fully premixed reaction reagent in any one of the preceding embodiments, the one-tube RT-qPCR fully premixed reaction system in any one of the preceding embodiments to amplify a to-be-detected sample according to the RT-qPCR method in the preceding embodiment to determine a viral origin of an amplification product.

Optionally, the virus includes at least one of norovirus, influenza A virus, influenza B virus, enteric conventional virus, Enterovirus 71 or Coxsackievirus 16.

Optionally, a source of the to-be-detected sample is selected from feces, vomit, a nasal swab, a throat swab, sputum, a bronchoalveolar lavage fluid, cells, chick embryo culture, a herpetic fluid, serum, a cerebrospinal fluid or tissue culture. Optionally, the source of the to-be-detected sample is selected from at least one of the feces, the vomit, the nasal swab, the throat swab, the sputum, the bronchoalveolar lavage fluid, the cells, the chick embryo culture, the herpetic fluid, the serum, the cerebrospinal fluid or the tissue culture.

Optionally, the throat swab includes a human throat swab or an avian throat swab.

The above sources of the to-be-detected sample can be selected by those skilled in the art according to actual requirements. For example, a sample such as feces or vomit of a patient can be selected as a to-be-detected sample of norovirus; a nasal swab, a throat swab, sputum or a bronchoalveolar lavage fluid of a patient or a sample such as cells ex vivo obtained through environmental sampling, chick embryo culture or an avian throat swab can be selected as a to-be-detected sample of influenza A virus or influenza B virus. A herpetic fluid, a throat secretion, feces, serum, a cerebrospinal fluid or tissue culture ex vivo of a patient can be selected as a to-be-detected sample of enteric conventional virus, Enterovirus 71 or Coxsackievirus 16.

For example, a certain specific amplification method includes the steps described below.

In S100, pretreatment is performed as follows: taking out the RT-qPCR fully premixed reaction system, melting the RT-qPCR fully premixed reaction system at room temperature, and thoroughly shaking, uniformly mixing and instantly centrifuging the RT-qPCR fully premixed reaction system.

In S200, sample addition is performed as follows: taking out an 8-Tube Strip for prepackaged containing the RT-qPCR full premixed reaction system, adding 10 µL each of the to-be-detected sample and the positive control and negative control nucleic acids, covering tube lids tightly, shaking uniformly and centrifuging instantly.

In S300, amplification and fluorescent signal detection are performed as follows: placing the PCR reaction tube obtained in step S200 in a fluorescence quantitative PCR instrument for the amplification and the fluorescent signal detection.

Optionally, according to the above RT-qPCR method, cycle parameters of the amplification and the fluorescent signal detection of the fluorescence quantitative PCR instrument in step S300 are shown in the following Table 1:

**Table 1**

| Step | | Temperature | Time | Number of Cycles |
|---|---|---|---|---|
| 1 | reverse transcription | 60 °C | 2 min | 1 cycle |
| 2 | predenaturation | 95 °C | 10 s | 1 cycle |
| 3 | denaturation | 95 °C | 1 s | 40 cycles |
| | annealing/extension/fluorescence detection | 60 °C | 10 s | |

The present disclosure provides a one-tube rapid RT-qPCR fully premixed reaction system. The present disclosure mainly aims at three problems in an existing RT-qPCR reaction system: (1) the total reaction system is divided into multiple tubes for storage, each component needs to be prepared and used in site each time, primer probes must be stored separately or together with an amplification reaction solution, and premixing each component in advance may lead to stability and specificity problems in the reaction system; (2) since the RT-qPCR reaction system is unable to adapt to a rapid amplification program and the reaction time is too long, the RT-qPCR reaction system is not applicable to rapid detection; (3) the adaptability of the RT-qPCR reaction system to the primer probes is insufficient so that a formula of the reaction system needs to be adjusted according to different primer probe combinations.

The present disclosure provides a one-tube RT-qPCR fully premixed reaction reagent. The reagent is used for preparing a one-tube RT-qPCR fully premixed reaction system for an RT-qPCR reaction to solve the problems that each component in an existing RT-qPCR reaction system is stored separately and needs to be prepared and used in site and the problems of decreased sensitivity, increased non-specificity, decreased storage stability and low applicability to primer probes after full premixing.

The present disclosure provides a fully premixed RT-qPCR reaction system and a use thereof. The hot start Taq DNA polymerase that supports ultra-fast extension is selected, thereby significantly reducing the operation time of the reaction system and controlling the entire reaction time within 30 min. Moreover, the reverse transcriptase that withstands a high temperature is selected, thereby improving the adaptability of the RT-qPCR reaction system to the primer probes and achieving the immediate insertion and use of different combinations of primer probes. The Taq enzyme monoclonal antibody with the double blocking ability (Double-Block anti-Taq DNA Polymerase Antibody) and the nucleic acid aptamer are added to the RT-qPCR reaction system, thereby ensuring that the enzymatic activity of the Taq DNA polymerase and the reverse transcriptase is effectively inhibited at a low temperature and the generation of primer dimers and a cutting effect of the Taq DNA polymerase on probes are reduced at a high temperature under pressure and long-term storage, significantly improving the specificity of the reaction system and avoiding false positive caused by the reaction system. The stabilizer and the enhancer are added to the RT-qPCR reaction system, thereby protecting the activity of the Taq DNA polymerase, the reverse transcriptase and other enzymes in the reaction system to the greatest extent, improving the stability of a high temperature, a pressure and long-term storage and having a better detection ability. The fully premixed RT-qPCR reaction system can be pressured at a high temperature of 37 °C for 7 days without an effect on the detection ability and can be stably stored below -20 °C for 12 to 15 months. Moreover, the reaction system can be directly subpackaged and used after the addition of nucleic acid so that a workload of an inspector is significantly reduced and the reaction system is particularly applicable to an automated platform.

### Example

Some embodiments of the present disclosure will be described in detail hereinafter in conjunction with the drawings. If not in collision, the embodiments described herein and the features thereof may be combined with each other.

### Example 1

A one-tube rapid RT-qPCR fully premixed reaction system in this example includes a 200 mmol/L Tris-HCl buffer solution (pH 8.4), 50 mmol/L KCl, 1.5 mmol/L MgCl₂, 0.25 mmol/L dN(U)TPs, 4 U Taq DNA polymerase, 6 U M-MLV reverse transcriptase, 0.25 U thermosensitive UNG enzyme, 2.5 U nuclease inhibitor, 0.5 µM upstream primer and downstream primer of norovirus and 0.55 µM probe. A sequence of a primer probe includes a sequence of the upstream primer of CTGGTGGATTGGAAATGTA (SEQ ID No.3), a sequence of the downstream primer of AATTCGGGCAGAAGATTG (SEQ ID No.4) and a sequence of a probe of CTGTCCACAATCCGAGGTCAT (SEQ ID No.5).

The 4U Taq DNA polymerase was a Taq DNA polymerase with an extension rate of 1.5 sec/kb.

### Example 2

This example differs from Example 1 in that the one-tube rapid RT-qPCR fully premixed reaction system includes a 200 mmol/L Tris-HCl buffer solution (pH 8.4), 50 mmol/L KCl, 1.5 mmol/L MgCl₂, 0.25 mmol/L dN(U)TPs, 4 U Taq DNA polymerase, 6 U M-MLV reverse transcriptase that withstands a high temperature, 0.25 U thermosensitive UNG enzyme, 2.5 U nuclease inhibitor and different combinations of primer probes.

The 6 U M-MLV reverse transcriptase was an M-MLV reverse transcriptase that withstands a high temperature of 60 °C.

### Example 3

This example differs from Example 2 in that the one-tube rapid RT-qPCR fully premixed reaction system includes a 200 mmol/L Tris-HCl buffer solution (pH 8.4), 50 mmol/L KCl, 1.5 mmol/L MgCl₂, 0.25 mmol/L dN(U)TPs, 4 U rapid Taq DNA polymerase, 6 U M-MLV reverse transcriptase, 0.25 U thermosensitive UNG enzyme, 2.5 U nuclease inhibitor, 5 ug/mL Double-Block anti-Taq DNA Polymerase Antibody, 0.5 µM upstream primer and downstream primer of norovirus and 0.55 µM probe. A sequence of a primer probe includes a sequence of the upstream primer of CTGGTGGATTGGAAATGTA (SEQ ID No.3), a sequence of the downstream primer of AATTCGGGCAGAAGATTG (SEQ ID No.4) and a sequence of a probe of CTGTCCACAATCCGAGGTCAT (SEQ ID No.5).

The Double-Block anti-Taq DNA Polymerase Antibody was a Taq enzyme monoclonal antibody with a double blocking ability, and a usage ratio of the antibody to the Taq DNA polymerase was 1:5 (content ratio: U/U).

### Example 4

This example differs from Example 3 in that the one-tube rapid RT-qPCR fully premixed reaction system includes a 200 mmol/L Tris-HCl buffer solution (pH 8.4), 50 mmol/L KCl, 1.5 mmol/L MgCl₂, 0.25 mmol/L dN(U)TPs, 4 U rapid Taq DNA polymerase, 6 U M-MLV reverse transcriptase, 0.25 U thermosensitive UNG enzyme, 2.5 U nuclease inhibitor, Double-Block anti-Taq DNA Polymerase Antibody, a 0.5 ng/mL nucleic acid aptamer, 0.5 µM upstream primer and downstream primer of norovirus and 0.55 µM probe. A sequence of a primer probe includes a sequence of the upstream primer of CTGGTGGATTGGAAATGTA (SEQ ID No.3), a sequence of the downstream primer of AATTCGGGCAGAAGATTG (SEQ ID No.4) and a sequence of a probe of CTGTCCACAATCCGAGGTCAT (SEQ ID No.5).

The nucleic acid aptamer was a single strand of an oligonucleotide of 50-100 bp.

### Example 5

This example differs from Example 4 in that the one-tube rapid RT-qPCR fully premixed reaction system includes a 200 mmol/L Tris-HCl buffer solution (pH 8.4), 50 mmol/L KCl, 1.5 mmol/L MgCl₂, 0.25 mmol/L dN(U)TPs, 4 U rapid Taq DNA polymerase, 6 U M-MLV reverse transcriptase, 0.25 U thermosensitive UNG enzyme, 2.5 U nuclease inhibitor, Double-Block anti-Taq DNA Polymerase Antibody, a 0.5 ng/mL nucleic acid aptamer, a PCR system stabilizer, 0.5 µM upstream primer and downstream primer of norovirus and 0.55 µM probe. A sequence of a primer probe includes a sequence of the upstream primer of CTGGTGGATTGGAAATGTA (SEQ ID No.3), a sequence of the downstream primer of AATTCGGGCAGAAGATTG (SEQ ID No.4) and a sequence of a probe of CTGTCCACAATCCGAGGTCAT (SEQ ID No.5).

The stabilizer includes 0.7 mg/mL BSA, 0.006% gelatin and 7.0% glycerol, where the percentages were volume percentages.

### Example 6

This example differs from Example 5 in that the one-tube rapid RT-qPCR fully premixed reaction system includes a 200 mmol/L Tris-HCl buffer solution (pH 8.4), 50 mmol/L KCl, 1.5 mmol/L MgCl₂, 0.25 mmol/L dN(U)TPs, 4 U rapid Taq DNA polymerase, 6 U M-MLV reverse transcriptase, 0.25 U thermosensitive UNG enzyme, 2.5 U nuclease inhibitor, Double-Block anti-Taq DNA Polymerase Antibody, a 0.5 ng/mL nucleic acid aptamer, a PCR system stabilizer, a PCR system enhancer, 0.5 µM upstream primer and downstream primer of norovirus and 0.55 µM probe. A sequence of a primer probe includes a sequence of the upstream primer of CTGGTGGATTGGAAATGTA (SEQ ID No.3), a sequence of the downstream primer of AATTCGGGCAGAAGATTG (SEQ ID No.4) and a sequence of a probe of CTGTCCACAATCCGAGGTCAT (SEQ ID No.5).

The enhancer includes 2% formamide, 2.5% DMSO, 0.2 M betaine, a 0.8 g/L SSB (single-stranded DNA binding protein), 0.2 M trehalose and 0.08 g/L sodium azide.

### Experimental Example

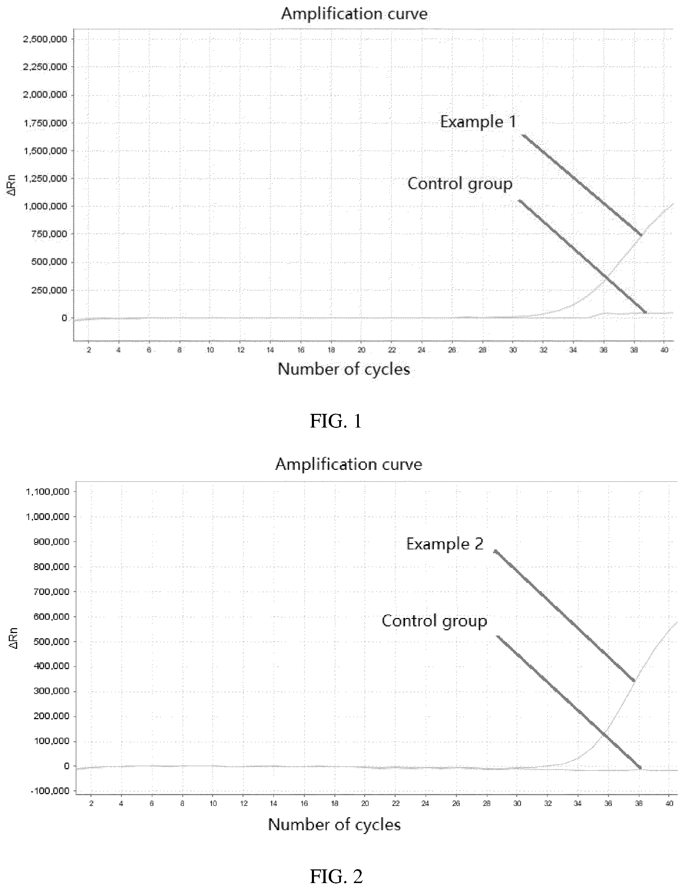

Reaction speed evaluation: 15 µL premixed reaction solution in Example 1 was taken and subpackaged, 5 µL extracted RNA/DNA (5000 copies/mL) was added as a template, and amplification and detection were performed according to the following RT-qPCR. A fully premixed RT-qPCR reaction system using conventional Taq DNA polymerases was used as a control group. The detection results are shown in FIG. 1.

An amplification program is shown in the following Table 2:

**Table 2**

| Step | | Temperature | Time | Number of Cycles |
|---|---|---|---|---|
| 1 | reverse transcription | 60 °C | 2 min | 1 cycle |
| 2 | predenaturation | 95 °C | 10s | 1 cycle |
| 3 | denaturation | 95 °C | 1s | 40 cycles |
| | annealing/extension/fluorescence detection | 60 °C | 10s | |

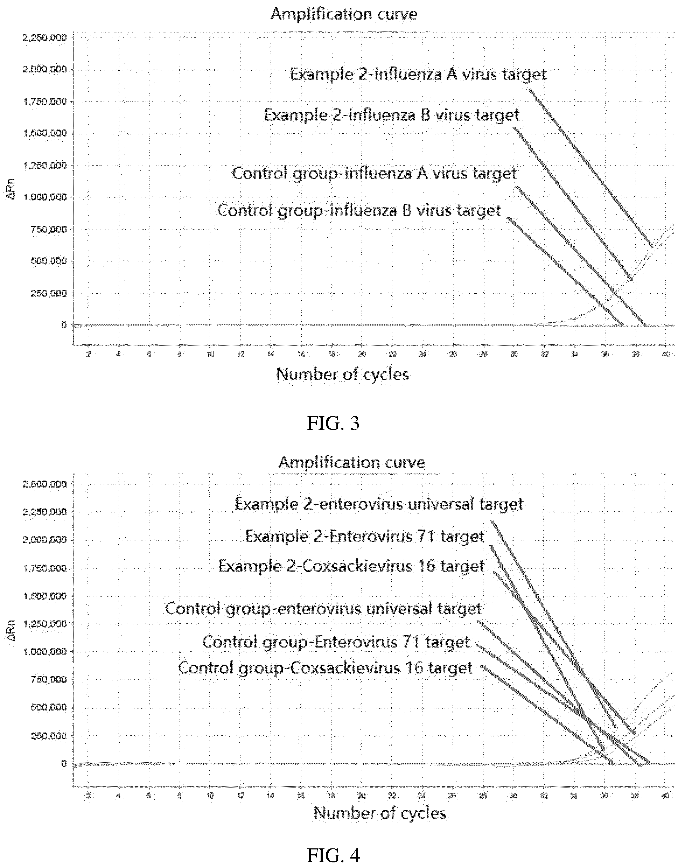

Applicability evaluation: 15 µL premixed reaction solution in Example 2 was taken and subpackaged, and 5 µL extracted RNA (1000 copies/mL) (a standard corresponding to a pathogen target) was added as a template; wherein a single primer probe was a primer probe of norovirus, which was consistent with that in Example 1; a double primer probe combination includes primer probes of influenza A virus and influenza B virus (a sequence of an upstream primer of influenza A virus was AGTCTTCTAACCGAGGTC (SEQ ID No.6), a sequence of a downstream primer of influenza A virus was TCGAGATCTGCGTTCTTC (SEQ ID No.7), a sequence of a probe of influenza A virus was CATCCTCAAGTCTCTGTGCGA (SEQ ID No.8), a sequence of an upstream primer of influenza B virus was GGAGTCTTATCCCAATTTG (SEQ ID No.9), a sequence of a downstream primer of influenza B virus was CTTCTGTGACCAGTCTAA (SEQ ID No.10), and a sequence of a probe of influenza B virus was CAAGAGCACCGATTATCACCAGAA (SEQ ID No.11)); a triple primer probe combination includes primer probes of enterovirus universal Enterovirus 71 and Coxsackievirus 16 (a sequence of a universal upstream primer of a enterovirus was GCAAGTAGTCACAGATTAG (SEQ ID No.12), a sequence of a universal downstream primer of the enterovirus was GATTCTTGTCACTAGCATT (SEQ ID No. 13), a sequence of a universal probe of the enterovirus was TACCAGCACTACAAGCCGC (SEQ ID No. 14), a sequence of an upstream primer of Enterovirus 71 was CACAGGTGAGCAGTCATC (SEQ ID No.15), a sequence of a downstream primer of Enterovirus 71 was CGCCCTACTGAAGAAACT (SEQ ID No.16), a sequence of a probe of Enterovirus 71 was TACAGGCAAGGTTCCAGCAC (SEQ ID No.17), a sequence of an upstream primer of Coxsackievirus 16 was CTTCCAGAGATTCGTTTG (SEQ ID No.18), a sequence of a downstream primer of Coxsackievirus 16 was CATATTATTCGGGCATTGA (SEQ ID No.19), and a sequence of a probe of Coxsackievirus 16 was CAGACAGCCACCAACCCAT (SEQ ID No.20)); and a fully premixed RT-qPCR reaction system using conventional M-MLV reverse transcriptases was used as a control group. Amplification and detection were performed according to the RT-qPCR program in Example 1. The detection results are shown in FIGS. 2 to 4.

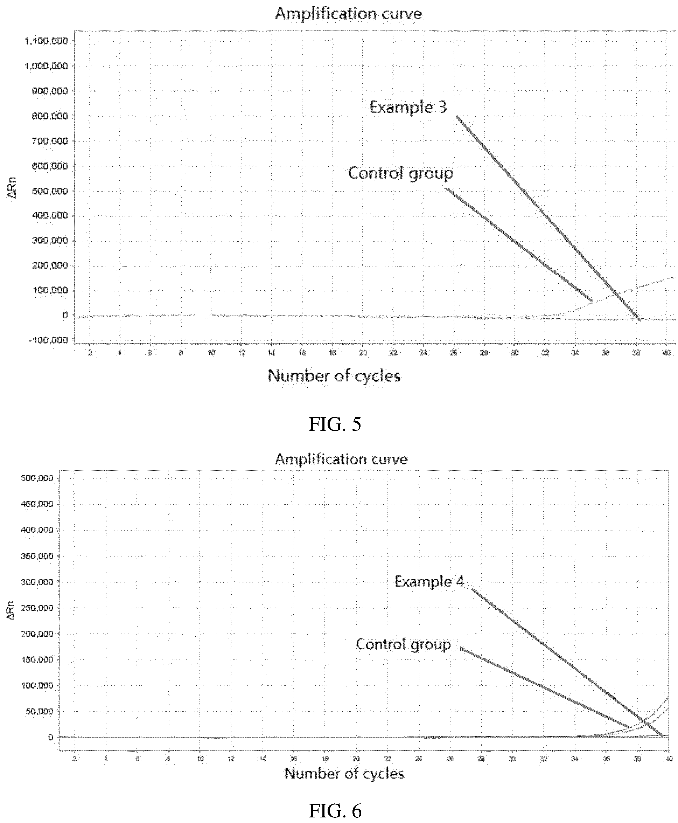

Specificity evaluation: 15 µL premixed reaction solution in Examples 3 and 4 was taken and subpackaged, 5 µL extracted human genomic nucleic acid (a negative sample) was added as a template, and the reaction solution was stored for 12 h at 2-8 °C after the sample addition and subjected to amplification and detection according to RT-qPCR. A fully premixed RT-qPCR reaction system without Double-Block anti-Taq DNA Polymerase Antibody and nucleic acid aptamers was used as a control group. The detection results are shown in FIGS. 5 and 6.

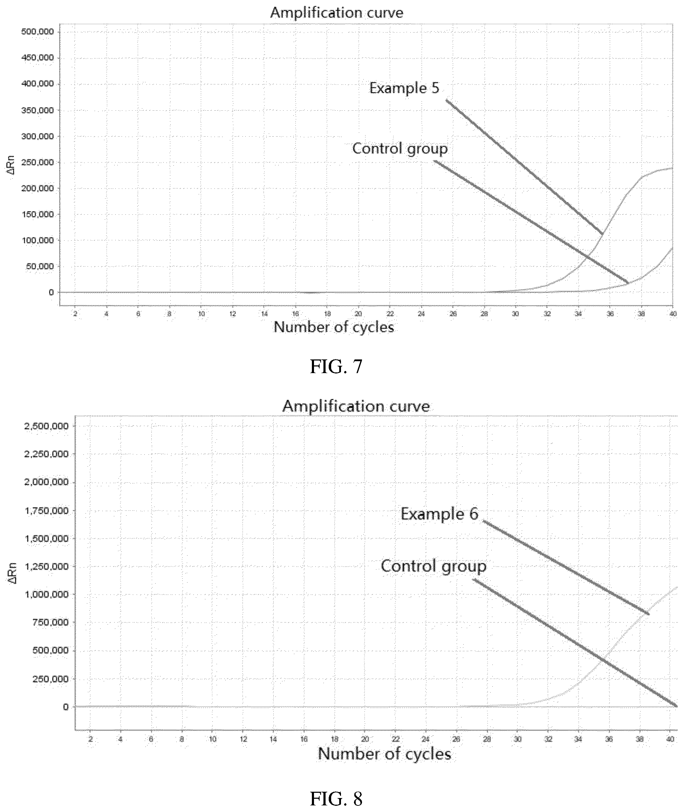

Stability evaluation: 15 µL premixed reaction solution in Example 5 was taken and subpackaged, 5 µL extracted RNA (5000 copies/mL) was added as a template, and the reaction solution was stored for 7 days at 37 °C after the sample addition and subjected to amplification and detection according to RT-qPCR. A fully premixed RT-PCR reaction system without a PCR stabilizer was used as a control group. Fluorescence quantitative PCR detection results are shown in FIG. 7.

Sensitivity evaluation: 15 µL premixed reaction solution in Example 6 was taken and subpackaged, 5 µL extracted RNA (400 copies/mL) was added as a template, and amplification and detection were performed according to RT-qPCR. A fully premixed RT-PCR reaction system without a PCR enhancer was used as a control group. Fluorescence quantitative PCR detection results are shown in FIG. 8.

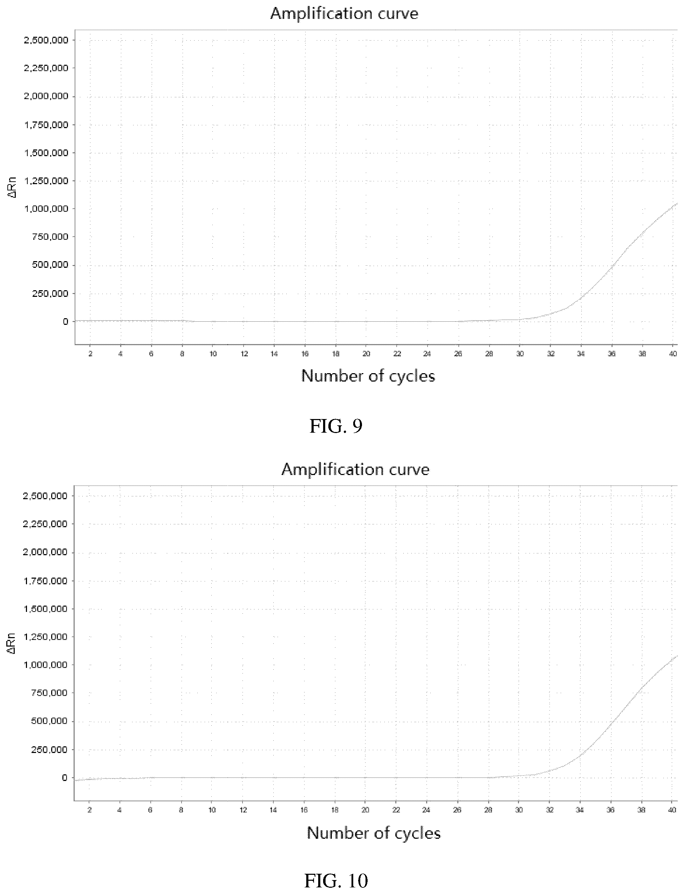

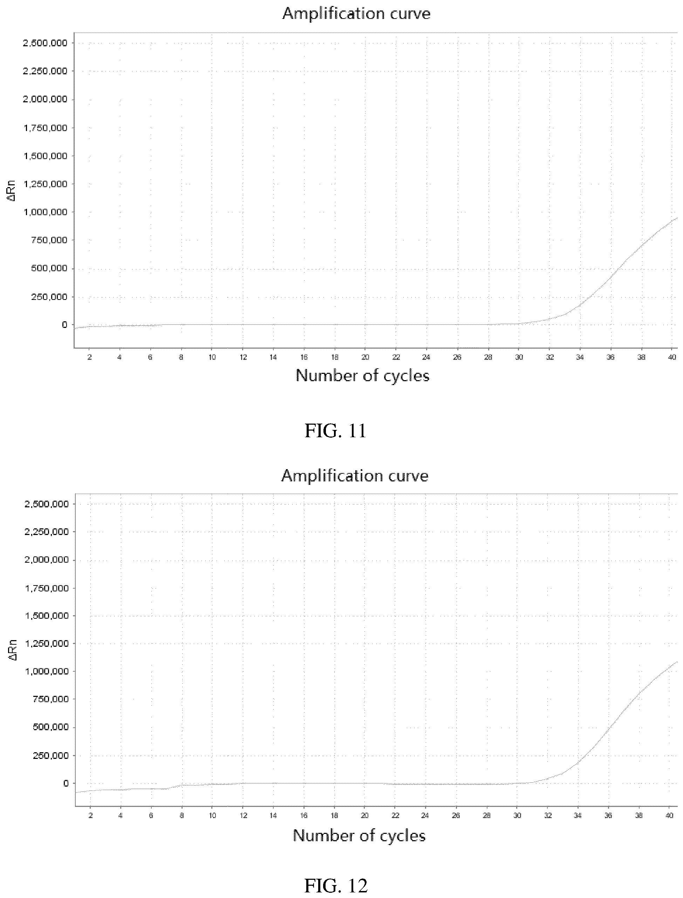

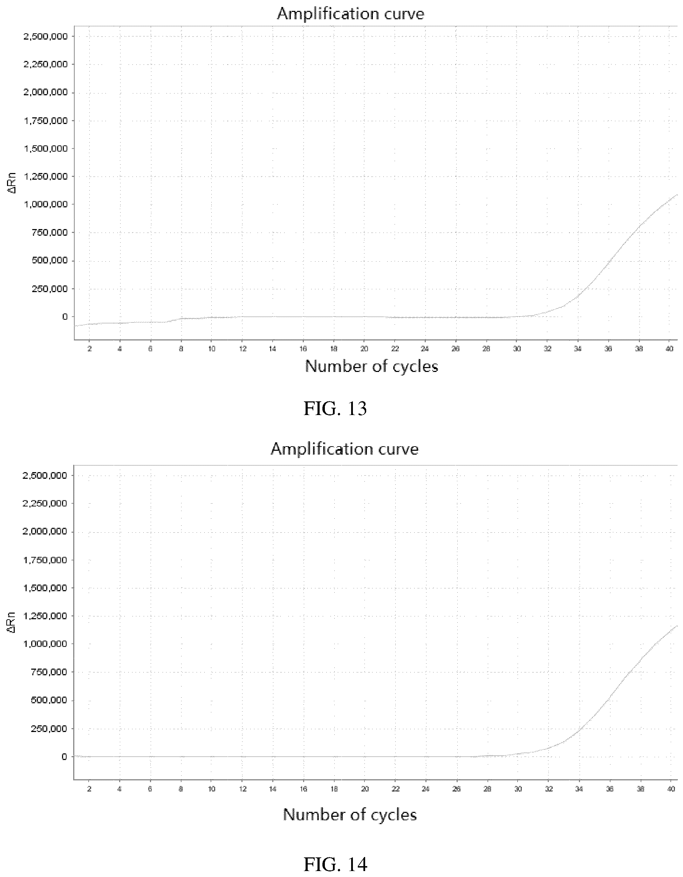

Long-term stability evaluation: the premixed reaction solution in Example 6 was tested when stored for 1 month at -20 °C, 3 months at -20 °C, 6 months at -20 °C, 9 months at -20 °C, 12 months at -20 °C and 15 months at -20 °C, where a specific experimental method was as follows: 15 µL of the solution after stored was taken and subpackaged, 5 µL extracted RNA (5000 copies/mL) was added as a template, and amplification and detection were performed according to RT-qPCR. A fully premixed RT-qPCR reaction system without Double-Block anti-Taq DNA Polymerase Antibody, nucleic acid aptamers, a PCR system stabilizer and a PCR system enhancer was used as a control group. The detection results are shown in FIGS. 9 to 14, respectively. FIGS. 9 to 14 correspond to the storage for 1 month at -20 °C, 3 months at -20 °C, 6 months at -20 °C, 9 months at -20 °C, 12 months at -20 °C and 15 months at -20 °C, respectively.

As can be seen from the above results, in the present disclosure, component screening, the preparation and use of the Double-Block anti-Taq DNA Polymerase Antibodies and the nucleic acid aptamers and the use of the PCR system stabilizers and the PCR system enhancers achieve the full premixing of each component in the RT-qPCR reaction system, an end customer can directly use the system without preparation, and the fully premixed system can be stored stably for a long time so that the stability and specificity problems of the long-term storage of the fully premixed system are solved; in the case of using the rapid DNA polymerases, the entire reaction time is shortened to less than 30 minutes so that the experimental time is significantly decreased and the detection throughput within the identical time is increased; in the case of using the reverse transcriptases that withstand a high temperature, the adaptability of the reaction system to the primer probes is significantly improved so that primer probes designed by different people can be directly used without the need for a secondary design.

Finally, it is to be noted that the above-mentioned embodiments are only used to explain the solutions of the present disclosure and shall not be construed as limitation thereto; although detailed descriptions have been made about the present disclosure by referring to the above-mentioned embodiments, those of ordinary skill in the art shall understood: modifications can be made on the solutions of the present application, or equivalent substitutions can be made on part or all of the technical features of the present application; such modifications or substitutions do not make the corresponding solution depart from the scope of the solutions of the embodiments in the present disclosure.

### INDUSTRIAL APPLICABILITY

The present disclosure provides a one-tube RT-qPCR fully premixed reaction reagent, a use and an RT-qPCR method. In the present disclosure, the reagent is used for preparing a one-tube RT-qPCR fully premixed reaction system for an RT-qPCR reaction to solve the problems that each component in an existing RT-qPCR reaction system is stored separately and needs to be prepared and used in site and the problems of decreased sensitivity, increased non-specificity, decreased storage stability and low applicability to primer probes after full premixing, thereby significantly reducing the operation time of the reaction system and improving the adaptability of the RT-qPCR reaction system to the primer probes. The fully premixed RT-qPCR reaction system of the present disclosure can be pressured at a high temperature of 37 °C for 7 days without an effect on the detection ability and can be stably stored below -20 °C for 12 to 15 months. Moreover, the reaction system can be directly subpackaged and used after the addition of nucleic acid so that a workload of an inspector is significantly reduced and the reaction system is particularly applicable to an automated platform. Therefore, the reaction system has excellent practical performance.

## Claims

1. A one-tube RT-qPCR fully premixed reaction reagent, comprising a Taq enzyme monoclonal antibody with a double blocking ability.

2. The one-tube RT-qPCR fully premixed reaction reagent according to claim 1, wherein an amino acid sequence of the Taq enzyme monoclonal antibody with the double blocking ability is shown in SEQ ID No.1 and SEQ ID No.2.

3. The one-tube RT-qPCR fully premixed reaction reagent according to claim 2, wherein a content ratio (U/U) of the Taq enzyme monoclonal antibody with the double blocking ability to a Taq DNA polymerase is 1:3-9, preferably 1:5.

4. The one-tube RT-qPCR fully premixed reaction reagent according to claim 1, further comprising a nucleic acid aptamer.

5. The one-tube RT-qPCR fully premixed reaction reagent according to claim 4, wherein a number of bases contained in the nucleic acid aptamer is 50-100 bp;
preferably, the nucleic acid aptamer has a concentration of 0.2-0.7 ng/mL, preferably 0.5 ng/mL.

6. The one-tube RT-qPCR fully premixed reaction reagent according to any one of claims 1 to 5, further comprising a stabilizer and/or an enhancer;
preferably, the stabilizer comprises at least two of 0.5-1 mg/mL BSA, 0.005% to 0.008% (v/v) gelatin, 5% to 10% (v/v) glycerol, or a 0.1% to 0.2% (v/v) non-ionic detergent;
preferably, the stabilizer comprises 0.7 mg/mL BSA, 0.006% (v/v) gelatin and 7% (v/v) glycerol;
preferably, the enhancer comprises 1% to 2% (v/v) formamide, 1% to 5% (v/v) DMSO, 0.1-0.3 M betaine, a 0.5-1.0 g/L SSB (single-stranded DNA binding protein), a 0.3-0.8 g/L pyrophosphatase, 0.1-0.3 M trehalose and 0.05-0.1 g/L sodium azide;
preferably, the enhancer comprises 2% formamide, 2.5% DMSO, 0.2 M betaine, a 0.8 g/L SSB (single-stranded DNA binding protein), 0.2 M trehalose and 0.08 g/L sodium azide.

7. Use of the one-tube RT-qPCR fully premixed reaction reagent according to any one of claims 1 to 6 for preparing a one-tube RT-qPCR fully premixed reaction system.

8. A one-tube RT-qPCR fully premixed reaction system, comprising the one-tube RT-qPCR fully premixed reaction reagent according to any one of claims 1 to 6;
further comprising a DNA polymerase, a reverse transcriptase, an UNG enzyme, an RNase inhibitor, a buffer, a stabilizer, an enhancer, upstream and downstream primers and a fluorescently labeled probe composition;
preferably, the DNA polymerase is a hot start Taq DNA polymerase with an extension rate of 1.5 sec/kb, and a preferred addition amount of 3-6 U;
preferably, the reverse transcriptase comprises an M-MLV reverse transcriptase with a tolerance temperature of more than or equal to 60 °C and an addition amount of 5-10 U;
preferably, the UNG enzyme is 0.1-0.5 U thermosensitive UNG enzyme;
preferably, an addition amount of the RNase inhibitor is 2-3 U;
preferably, the buffer is a 100-500 mmol/L Tris-HCl buffer solution;
preferably, the buffer contains an inorganic salt, wherein the inorganic salt is a sulfate and/or a chloride salt, more preferably the chloride salt.

9. The one-tube RT-qPCR fully premixed reaction system according to claim 8, wherein the upstream and downstream primers and fluorescently labeled probe composition comprise a single detection composition, a double detection composition, a triple detection composition or a quadruple detection composition based on a difference in a number of types of a nucleic acid product obtained after amplification in a single time;
preferably, the nucleic acid product is derived from a virus;
preferably, the virus is selected from at least one of norovirus, influenza A virus, influenza B virus, enteric conventional virus, Enterovirus 71 or Coxsackievirus 16;
preferably, the single detection composition is a primer probe composition of norovirus;
preferably, a nucleotide sequence of an upstream primer in the primer probe composition of norovirus is shown in SEQ ID No.3, a nucleotide sequence of a downstream primer in the primer probe composition of norovirus is shown in SEQ ID No.4, and a nucleotide sequence of a probe in the primer probe composition of norovirus is shown in SEQ ID No.5;
preferably, the double detection composition is a primer probe composition of influenza A virus and influenza B virus;
preferably, in the primer probe composition of influenza A virus and influenza B virus, a nucleotide sequence of an upstream primer of influenza A virus is shown in SEQ ID No.6, a nucleotide sequence of a downstream primer of influenza A virus is shown in SEQ ID No.7, a nucleotide sequence of a probe of influenza A virus is shown in SEQ ID No.8, a nucleotide sequence of an upstream primer of influenza B virus is shown in SEQ ID No.9, a nucleotide sequence of a downstream primer of influenza B virus is shown in SEQ ID No.10, and a nucleotide sequence of a probe of influenza B virus is shown in SEQ ID No.11;
preferably, the triple detection composition is a primer probe composition of enteric conventional virus, Enterovirus 71 and Coxsackievirus 16;
preferably, the primer probe composition of enteric conventional virus, Enterovirus 71 and Coxsackievirus 16 contains:
a universal upstream primer of enteric conventional virus with a nucleotide sequence shown in SEQ ID No.12, a universal downstream primer of enteric conventional virus with a nucleotide sequence shown in SEQ ID No.13 and a universal probe of enteric conventional virus with a nucleotide sequence shown in SEQ ID No.14;
an upstream primer of Enterovirus 71 with a nucleotide sequence shown in SEQ ID No.15, a downstream primer of Enterovirus 71 with a nucleotide sequence shown in SEQ ID No.16 and a probe of Enterovirus 71 with a nucleotide sequence shown in SEQ ID No.17; and
an upstream primer of Coxsackievirus 16 with a nucleotide sequence shown in SEQ ID No.18, a downstream primer of Coxsackievirus 16 with a nucleotide sequence shown in SEQ ID No.19 and a probe of Coxsackievirus 16 with a nucleotide sequence shown in SEQ ID No.20.

10. An RT-qPCR method, comprising: amplifying a to-be-detected sample by using the one-tube RT-qPCR fully premixed reaction system of claim 8 or 9, according to the following program: reverse transcription for 1-5 min at 55-65 °C, predenaturation for 5-15s at 94-98 °C, denaturation for 1-5s at 94-98 °C, annealing and extension for 8-15s at 55-65 °C, and 38 to 45 cycles of denaturation, annealing and extension; and
preferably, the program of amplification comprises: reverse transcription for 2 min at 60 °C, predenaturation for 10s at 95 °C, denaturation for 1s at 95 °C, annealing and extension for 10s at 60 °C, and 40 cycles of denaturation, annealing and extension.

11. Use of the one-tube RT-qPCR fully premixed reaction reagent according to any one of claims 1 to 6, or the one-tube RT-qPCR fully premixed reaction system according to claim 8 or 9 for viral infection diagnosis.

12. The use according to claim 11, wherein the virus comprises at least one of norovirus, influenza A virus, influenza B virus, enteric conventional virus, Enterovirus 71 or Coxsackievirus 16.

13. A method for diagnosing a disease related to viral infection in a subject, comprising:
(A) performing amplification on a sample from the subject through an RT-qPCR method under a condition sufficient for a binding reaction to occur, with the one-tube RT-qPCR fully premixed reaction reagent according to any one of claims 1 to 6 or the one-tube RT-qPCR fully premixed reaction system according to claim 8 or 9; and
(B) determining a viral origin of an amplification product.

14. The method according to claim 13, wherein the virus comprises at least one of norovirus, influenza A virus, influenza B virus, enteric conventional virus, Enterovirus 71 or Coxsackievirus 16.

15. The method according to claim 13 or 14, wherein a source of the sample is selected from feces, vomit, a nasal swab, a throat swab, sputum, a bronchoalveolar lavage fluid, cells, chick embryo culture, a herpetic fluid, serum, a cerebrospinal fluid or tissue culture.
